# EUROPEAN PATENT APPLICATION

(11) **EP 4 311 572 A1**
(43) Date of publication of application: **31.01.2024**
(21) Application number: 22208205.9
(22) Date of filing: 18.11.2022
(51) Int. Cl.: A61N 1/32, A61F 7/00, A61N 5/06, A61M 11/00, A61H 23/02

(54) **SKIN CARE METHOD OF SKIN BEAUTY DEVICE**

(30) Priority: 29.07.2022 KR 20220094510
(71) Applicant: Soovon Co., Ltd., Incheon 21315 (KR)
(72) Inventor: OH, Kyoung Hee, 10388 Goyang-si, Gyeonggi-do (KR)
(74) Representative: Isarpatent

(57) **Abstract**

The skin care method of skin beauty device according to the present invention can lower the skin barrier by charging negative potential to skin through mist spraying, expand pores and reproduce the skin through temperature stimulation and irradiation of red light after cleaning wastes from inside the pores, and more effectively enable cosmetics to be introduced into the skin through EP function and vibration function. Furthermore, the skin care procedures can be finished by implementing the pore shrinkage through cooling stimulation and antimicrobial action through blue light irradiation. The more efficient skin care can be realized through adequate implementation of thus-mentioned series of skin care functions according to the order, compared to individual implementations of various functions of the skin beauty device, whereby users not accustomed to the skin care can easily care the skin.

## Description

### [CROSS-REFERENCE TO RELATED APPLICATION]

This application claims priority to and the benefit of Korean Patent Application No. 10-2022-0094510 filed on July 29, 2022 in the Korean Intellectual Property Office, the entire content of which is incorporated herein by reference.

### [Technical Field]

The teachings in accordance with exemplary and non-limiting embodiments of this invention generally relate to a method implementing skin care using a skin beauty device, and more particularly, to a skin care method of skin beauty device configured to allow cosmetics to be well penetrated into a user's skin by adequately operating a skin beauty device integrated with various functions, whereby skin care can be more efficiently realized.

### [Background Art of the Invention]

These days, females as well as males pay special attention to beauty to keep their good looks. Generally, nutrition, nourishment, collagen to help boost skin function, nutritional fluid and cosmetic products including, but not limited to, essence are widely used for beauty.

Recently, various beauty devices that help users' skin care are released and used.

A skin beauty device can implement various skin care functions including, but not limited to, function to spray microscopic state of fine mists, function to provide temperature stimulations, EP (Electroporation) function and physical massage function.

Particularly, the Korean registered patent No.: 10-2342012, entitled skin beauty device can provide, as one portable device, hydrogen sterilized water spray function, temperature stimulation function, EP function, and photo-stimulating function to allow managing more convenient, simple and efficient skin care.

However, it would be difficult to efficiently implement the skin care when respective skin care functions are individually utilized.

In order to cause mutually synergistic effects shown through each skin care function, the said each skin care function must be implemented in adequate order in response to characteristics thereof, but it is difficult for a user to know the adequate skin care method in detail and apply the same like a professional.

Therefore, even though the skin beauty devices are developed to implement various functions, needs are great to allow an efficient skin care to be implemented by correctly and adequately applying each function.

### [Related Technical Document]

### [Patent Document]

Korean Registered Patent No. 10-2342012 (Published date: Dec. 23, 2021: Title: Multi-functional integrated skin beauty device)

### [Summary of the Invention]

### [Technical Subject]

The present invention is therefore devised to cater to the abovementioned needs, and it is an object of the present invention to provide to skin care method of skin beauty device configured to allow implementing an efficient skin care by adequately and correctly applying various functions of skin care device.

### [Technical Solution]

In one general aspect of the present invention, there may be provided a skin care method of skin beauty device, the method implemented by the skin care device including a mist processing unit that sprays sterilized water in a mist type by generating the sterilized water through water electrolysis, a temperature stimulation processing unit applying a temperature stimulation, an EP processing unit applying an electric signal to an electroporation electrode, a power supply unit supplying an electric power, an input unit receiving an operation command and a controller performing a control in response to the operation command inputted through the input unit, the method comprising:
controlling in such a manner that the mist processing unit sprays the mist for a predetermined time (first step):
controlling in such a manner that the temperature stimulation processing unit applies heating (thermal) stimulation for a predetermined time (second step); controlling in such a manner that the EP processing unit applies an electric signal to an electroporation electrode for a predetermined time (third step);
and controlling in such a manner that the temperature stimulation processing unit applies a cooling stimulation for a predetermined time (fourth step).

Preferably, the skin beauty device may further comprise a light processing unit outputting a light for skin care.

At this time, the second step may be so configured as to allow the light processing unit to apply a red light in a particular wavelength band from at least a part of the period in which the heating stimulation is applied.

Furthermore, the fourth step may be so configured as to allow the light processing unit to apply a blue light in a particular wavelength band from at least a part of the period in which the heating stimulation is applied.

Preferably, the skin beauty device may further comprise a vibration processing unit applying a vibration stimulation to the skin.

At this time, the third step may be so configured as to allow the vibration processing unit to apply vibrations to the skin from at least a part of the period in which an electric signal is applied to the electroporation electrode.

Preferably, the mist processing unit may be so configured as to spray the mist having a diameter smaller than 5nm in order to push out wastes by penetrating into pores.

### [Advantageous Effects]

Teachings in accordance with the exemplary embodiments of this invention may have an advantageous effect in that mist spray, heating (thermal) stimulation application, electroporation function implementation, and cooling (cold) stimulation application are realized in sequence.

Another advantageous effect may be that skin is charged with negative potential through mist spray to thereby clean up wastes inside pores by reducing the skin barriers.

Still another advantageous effect may be that pores are expanded and skin reproductions are promoted through heating stimulations and red light irradiations, whereby the EP functions are implemented to allow cosmetics to be more effectively penetrated into skin.

Thereafter, pore shrinkages caused by cooling stimulation and antibacterial activities through blue light irradiation may be realized to allow nutrients to be kept inside the skin for as long as possible.

Because of these series of processes being implemented in sequence, the skin care can be more effectively realized than various functions of skin beauty device being implemented individually, whereby even users who are not familiar with skin care can easily and correctly manage her or his skin care.

### [Brief Description of Drawings]

FIG. 1 illustrates a skin care method of skin beauty device being implemented according to an exemplary embodiment of the present invention,
FIG. 2 illustrates an example of the temperature stimulation, electropolation stimulation, light stimulation and vibration stimulation being intensively implemented on one surface contacting the skin
FIGS. 3, 4 and 5 illustrate an example of a housing of a skin beauty device.
FIG. 6 illustrates in detail a mist processing unit and temperature stimulation processing unit respectively arranged on both sides of head unit of housing according to an exemplary embodiment of the present invention,
FIG. 7 illustrates in detail a mist processing unit according to an exemplary embodiment of the present invention,
FIG. 8 illustrates in detail the arrangement of skin contact member, transparent window and electroporation electrode according to an exemplary embodiment of the present invention,
FIG. 9 illustrates in detail a temperature stimulation processing unit according to an exemplary embodiment of the present invention,
FIG. 10 illustrates a first heat dissipation unit directly contacting the sterilized water inside a water tank according to an exemplary embodiment of the present invention,
FIG. 11 illustrates a cradle unit according to an exemplary embodiment of the present invention,
FIG. 12 illustrates a skin care method according to an exemplary embodiment of the present invention,
FIG. 13 illustrates wastes inside pores being discharged in response to mist spray according to an exemplary embodiment of the present invention,
FIG. 14 illustrates an example explaining a skin care effect realized in step 2, and
FIG. 15 illustrates an example explaining a skin care effect realized in step 4.

### [Detailed Description]

The present invention may be embodied in many different modifications and alterations and have several exemplary embodiments, where specific exemplary embodiments will be exemplified through drawings which are to be described in details in the detailed descriptions. However, it should be appreciated that the present invention is not intended to be limited to particular exemplary embodiments but to encompass all equivalent alterations and/or modifications within the scope and spirit of the present invention.

In describing the present invention, detailed descriptions of constructions or processes known in the art may be omitted to avoid obscuring appreciation of the invention with unnecessary detail regarding such known constructions and functions. The terms used in the present invention are used simply to explain particular exemplary embodiments and are not intended to be limited to particular exemplary embodiments. It is to be understood that the singular forms include plural referents unless the context clearly dictates otherwise.

The terms "comprises," "comprising," "including," and "having," in the present invention are inclusive and therefore specify the presence of stated features, integers, steps, operations, elements, and/or components, but do not preclude the presence or addition of one or more other features, integers, steps, operations, elements, components, and/or groups thereof.

Although the terms first, second, third, etc. may be used herein to describe various elements, these elements should not be limited by these terms. These terms may be only used to distinguish one element from another element.

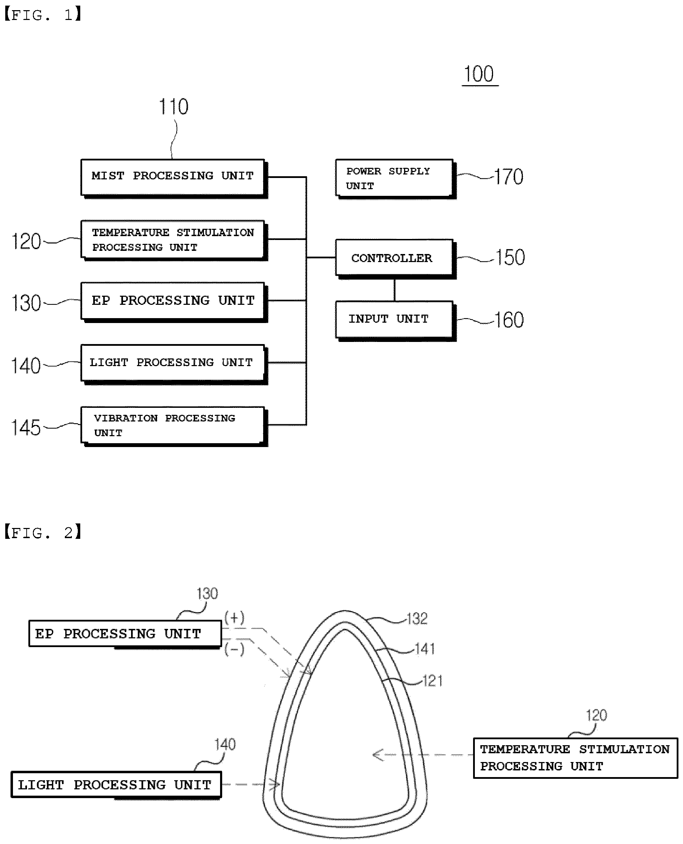

FIG. 1 illustrates a skin care method of skin beauty device 100 being implemented according to an exemplary embodiment of the present invention, where the device 100 may be formed by including a mist processing unit 110 that sprays sterilized water in a mist type by generating the sterilized water through water electrolysis, a temperature stimulation processing unit 120 applying a temperature stimulation, an EP processing unit 130 applying an electric signal to an electroporation electrode, an input unit 160 receiving an operation command, a power supply unit 170 supplying an electric power, and a controller 150 performing an overall control of the skin beauty device 100 in response to the operation command inputted through the input unit 160.

Furthermore, the skin beauty device 100 may be formed by further including a light processing unit 140 outputting a light for skin care and a vibration processing unit 145 applying a vibration stimulation for skin massage.

The input unit 160 may receive various commands from a user about operations of the skin beauty device 100. The operation commands inputted through the input unit 160 and input method may be variably constituted but the present invention is not particularly limited thereto.

For example, the input unit 160 may include various switches and buttons related to various functions including, but not limited to, power ON/OFF of skin beauty device 100, ON/OFF of mist function, ON/OFF of temperature stimulation function, ON/OFF of electroporation function, ON/OFF of light stimulation function, ON/OFF of massage vibration function, light type set-up, cooling stimulation temperature set-up, and heating stimulation temperature set-up.

Furthermore, the input unit 160 may be so configured as to individually control various functions provided by the skin beauty device 100 and to receive commands to allow a series of various skin care functions to be continuously implemented in response to the skin care method according to the present invention.

The EP processing unit 130 may increase permeability of skin cell membranes by applying an electric field to the skin cell membranes through application of an electric signal to an electroporation electrode.

The voltages, waveforms and frequencies applied by the EP processing unit 130 to the electroporation electrode may be variously configured as needed, and types, structures, arrangements of electroporation electrode may be also variably configured.

The light processing unit 140 may output one or more types of skin care lights capable of being used for skin care, and may simultaneously or selectively output lights of various wavelength bands (i.e.,: 400~470nm, 570~590nm, 630~700nm, 800~1,200nm, etc.).

The light processing unit 140 may output a skin care light through an LED (Light-Emitting Diode), and the wavelengths, intensities and patterns of the light may be variably formed.

The light outputted from the light processing unit 140 may show various skin care effects including, but not limited to, antioxidant, skin lightening, skin tone-up, wrinkle recovery, soothing of sensitive skin, acne improvement, blemish/pigmentation suppression, soothing of skin, strengthening of skin elasticity, improvement of blood circulation, pore shrinkage, senescence inhibition, and skin regeneration, depending on characteristics thereof.

Structure for temperature stimulation, structure for EP electrode for EP stimulation, a light output structure for light stimulation and structure for applying vibration stimulation to skin by generating vibrations for massage may be variably configured.

Hereinafter, although explanations will be given with reference to the present inventor-invented multi-functional integrated skin beauty device under Korean Registered Patent No.:10-2342012 as a detailed example of skin care device 100 for implementing the skin care according to the present invention, the skin beauty device 100 for implementing the skin care method according to the present invention will not be limited thereto and may be formed with various types, structures and functions.

The skin beauty device 100 may be so configured as to provide the temperature stimulation function, the EP function, the light stimulation function and the massage vibration function intensively on one surface contacted by the skin. As a result, each skin stimulation can be intensively realized to further enhance the skin care effect.

Referring to FIG. 2, the temperature stimulation processing unit 120 may be so configured as to apply the temperature stimulation to skin using a thermoelement and may be formed by including a skin contact member 121 which is an element heated or cooled by being directly contacted to a user's skin.

The said skin contact member 121 may be disposed on one surface of a housing of the skin beauty device 100 contacting the user's skin.

Furthermore, a transparent window 141 may be disposed along the skin contact member 121 to allow emitting a light outputted from the light processing unit 140, and an outer electrode member 132 may be disposed along an outer surrounding of the transparent window 141.

At this time, the skin contact member 121 and the outer electrode member 132 may be formed with an electrically conductive material, and the skin contact member 121 and the outer electrode member 132 may form the EP electrode.

In other words, the skin contact member 121 may be a positive (+) or negative (-) electrode for EP stimulation in addition to the role of providing a temperature stimulation to the skin, and the outer electrode member 132 may be an electrode having an opposite polarity (negative pole or positive pole) of the skin contact member 121.

Furthermore, the vibration processing unit 145 may be so disposed as to vibrate the skin contact member 121 in order to provide the massage vibration function, but the present invention is not limited thereto.

Although FIG. 2 illustrates an example where the skin contact member 121, the transparent window 141 and the outer electrode member 132 are formed with an approximately triangular shape, the material, shape and the arrangement of the skin contact member 121, the transparent window 141 and the outer electrode member 132 may be variably formed, and the present invention is not limited thereto.

For example, the skin contact member 121, the transparent window 141 and the outer electrode member 132 may be formed with arbitrary polygonal shapes including a round shape and a square shape, and an oval shape, but may not be formed with the same shape.

As one implementing method to allow integrally providing each function from one surface and raising mutual synergism, the transparent window 141 may be formed with a ring shape that surrounds an outer surface of the skin contact member 121.

Furthermore, the outer electrode member 132 formed along the outer surface of transparent window 141 may be also formed with a ring shape encompassing the transparent window 141.

The housing forming an outer look of the skin beauty device 100 may be organized in a variety of ways, but it would be preferable that the housing be formed with a portable shape, whereby a user can use and grapple the housing with one hand.

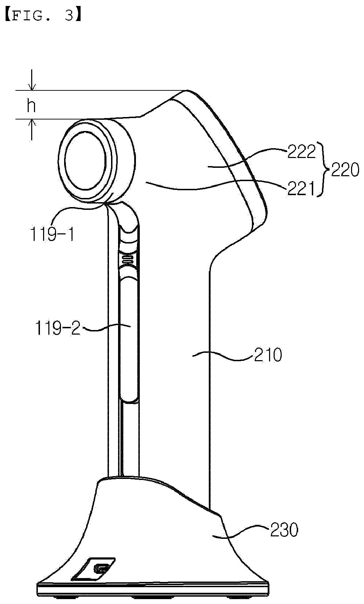

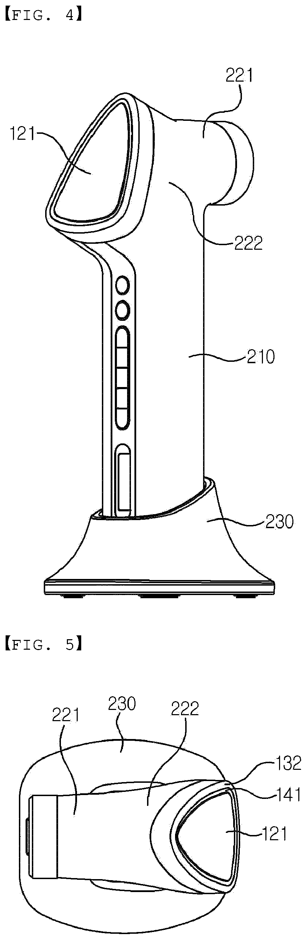

FIGS. 3, 4 and 5 illustrate an example of a housing portably forming a skin beauty device 100, where FIG. 3 is a perspective view viewed from a direction where mists are spouted, FIG. 4 is a perspective view viewed from a direction where the skin contact member 121 that contacts a user's skin is visible in order to provide a temperature stimulation, and FIG. 5 is a view seen from above a housing.

The said housing may be formed by including a pillar-shaped handle part 210 graspable by one hand of a user, and a head part 220 protruding from an upper end of the handle part 210 to both lateral surfaces. At this time, the head part 220 may include a first head part 221 protruding from an upper end of the handle part 210 to one lateral surface, and a second head part 222 protruding to an opposite lateral surface thereof.

The position (height) and angle of the first head part 221 and the second head part 222 protruding from the handle part 210 may be variably formed. Furthermore, shapes of the first head part 221 and the second head part 222 may be also variably configured.

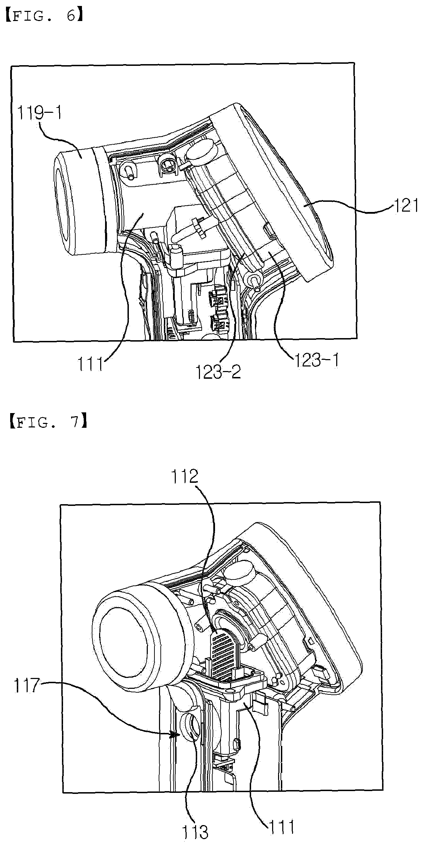

Referring to FIG. 6, the first head part 221 of the housing may be disposed with respective elements for mist processing, and the second head part 222 protruding to the other lateral surface may be disposed with respective elements for temperature stimulation processing.

A distal end surface of the first head part 221 may be formed with a raw water inlet to allow water to be poured into a water tank 111 of the mist processing unit 110, and a distal end surface of the second head part 222 may be formed with a skin contact member 121 that applies the temperature stimulation.

That is, the first head part 221 may be related to raw water supply for mist generation and water tank space arrangement, and the second head part 222 may be a part to provide stimulation by being contacted to skin.

Meantime, when the first head part 221 is arranged at a height same as that of the second head part 222, or at a height higher than the second head part 222, there may be generated inconveniences in the course of using the second head part 222.

For example, the process of stimulating a facial skin using the second head part 222 may be disturbed because the first head part 221 is hitched at the bridge of a nose.

In relation thereto, as shown in FIG. 3, an extreme upper end of the second head part 222 may be configured to be higher than the first head part 221 at a predetermined length (h).

Then, the inconveniences that may be generated due to the first head part 221 may be prevented when the temperature stimulation, EP stimulation, light stimulation and vibration stimulation are applied to the skin through the distal end surface of the second head part 222. As shown in the drawing, when the distal end surface of the second head part 222 is configured with a triangular shape, the process of caring for the facial skin including the bridge of the nose using the second head part 222 may become more advantageously convenient.

Furthermore, when the size of the distal end surface of the second head part 222 is configured to be larger than that of the first head part 221, various functions realized through the distal end surface of the second head part 222 may be more conveniently utilized. Although the example shown in the drawing has indicated that the second head part 222 starts to be protruded from the handle part 210 ahead of the first head part 221 to allow being more broadly formed than the first head part 221, the present invention is not limited thereto.

Referring to FIGS. 6 and 7, the mist processing unit (110) may be formed by including a water tank 111 that contains the raw water such as tap water, electrode modules 112 disposed inside the water tank 111 to electrolyze the raw water and convert the electrolyzed water to sterilized water, an ultrasonic vibrator (oscillator) 113 generating a fine particulate mist by applying vibration to the sterilized water generated from the electrode modules 112, and a mist outlet 117 from which the mist generated by the ultrasonic vibrator 113 is discharged.

The structures, shapes and materials of water tank 111 may be variously configured if necessary, but the present invention is not particularly limited thereto.

The distal end surface of first head part 221 may be disposed with a raw water inlet to pour water into the water tank 111 of the mist processing unit 110, and the water tank 111 may be formed with an approximate '¬' shape across an upper space of the handle part 210 from the raw water inlet.

The raw water inlet formed on a distal end surface of first head part 221 may be opened or closed through an inlet cap 119-1, where a user may shut off and tightly close the inlet cap 119-1 after the user opens the inlet cap 119-1 and pours the raw water into the water tank 111.

Furthermore, an upper end of the handle part 210 may be formed with a mist outlet 117 to a direction where the raw water inlet is formed.

The electrode modules 112 may be disposed by being mutually spaced apart, and may be formed by including a pair or more pairs of electrode plates each connected to the negative (-) and positive (+) pole. and generate sterilized water having the sterilizing power by disinfecting or sterilizing the water molecules using the principle of Underwater Plasma.

In other words, the sterilizing power may be generated by allowing creating various negative ions (O⁻, O₃⁻, OH⁻, HOCl, H₂O₂) in the underwater through two electrode plates in response to plasma discharge. The electrode modules 112 may be variously formed.

The ultrasonic vibrator 113 may be so disposed with such a shape as to encompass a surrounding of the mist outlet 117 to efficiently spout the mists.

As one example, when the mist outlet 117 is formed with a round shape, the ultrasonic vibrator 113 may be formed with a ring shape encompassing the surrounding of the mist outlet 117.

With reference to generation of mists using the ultrasonic vibrator 113, the shape of water tank 111 at a part where the ultrasonic vibrator 113 is disposed may be such that an anteroposterior length is as narrow as possible and a length from side to side is formed to be similar to an area of the ultrasonic vibrator 113.

Here, the anteroposterior length refers to a length between a surface where the ultrasonic vibrator 113 is disposed and an opposite surface thereof, whereby the mist spray efficiency through the ultrasonic vibrator 113 may be improved.

For example, the sterilizing water can be sufficiently supplied to the ultrasonic vibrator 113 for as long time as possible, when the sterilizing water is insufficient. An area contacted by the ultrasonic vibrator 113 and the sterilizing water may be reduced because the sterilizing water is spread broadly as the water tank 111 grows broader at a part where the ultrasonic vibrator 113 is disposed.

Furthermore, the sterilizing water contained at a lower level than a lowermost bottom position of the ultrasonic vibrator 113 may be difficult in mist conversion, such that the bottom position of the water tank 111 may be disposed adjacent to the lowermost bottom position of the ultrasonic vibrator 113.

Then, the area contacted by the sterilizing water with the ultrasonic vibrator 113 may be broadly maintained for a long time during a course of the sterilizing water being reduced.

When an electric power is applied to the electrode modules 112 in response to the control of the controller 150, the electrolysis is realized to allow the raw water to be converted to sterilizing water, whereby the sterilizing water mists are generated by the ultrasonic vibrator 113 to be sprayed through the mist outlet 117.

FIG. 3 illustrates an example where a switch for turning on/off the mist function is disposed as an up/down sliding button 119-2 underneath the mist outlet 117.

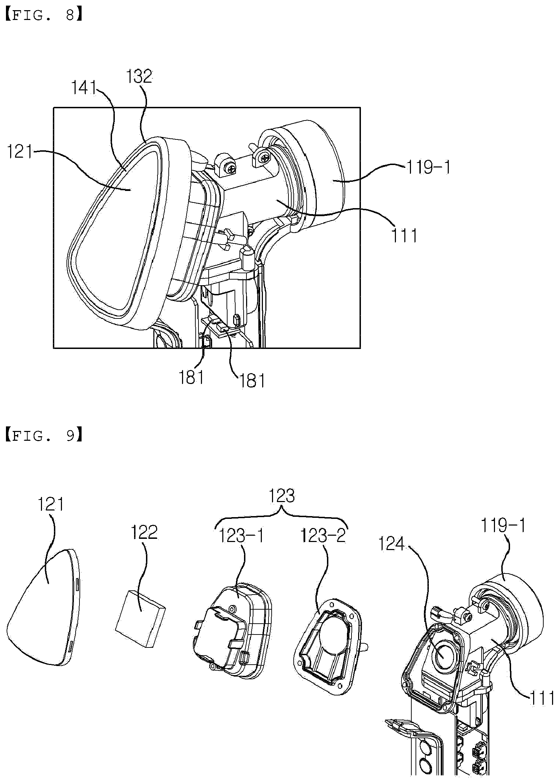

Referring to FIGS. 8, 9 and 10, the temperature stimulation function, the EP function, the light stimulation function and the massage vibration function and other functions may be intensively provided from a distal end surface of the second head part 222.

The shapes, structures and materials of skin contact member 121 may be variably formed.

For example, the skin contact member 121 may be formed with a material applicable with an electric signal in order to provide an EP stimulation along with having adequate heat transfer properties. A structural example of the skin contact member 121 formed with a triangular plate type is illustrated but the present invention is not limited thereto.

A transparent window 141 may be disposed to emit a light from a light processing unit 140 along a surrounding of the skin contact member 121, and one or more LED elements forming the light processing unit 140 may be disposed inside the transparent window 141. Furthermore, an outer electrode member 132 may be disposed along an outer surrounding of the transparent window 141.

The temperature stimulation processing unit 120 may be formed by including a skin contact member 121 to apply a temperature stimulation by being contacted with a user's skin, and a heat transfer element 122 to heat or cool the skin contact member 121.

The heat transfer element 122 for temperature adjustment may be variably formed and may be formed by particularly using a Peltier element.

One surface of the Peltier element 122 may be so configured as to allow directly performing the role of the skin contact member 121. One surface (outside surface) of the Peltier element 122 contacting the skin contact member 121 may be treated with a particular temperature to stimulate a user's skin. In other words, when a current is applied to the Peltier element 122 in response to the control of the controller (150), a relevant surface of the Peltier element 122 may be heated or cooled along a direction of the applied current.

Particularly, an inside surface of the Peltier element 122 must be able to be adequately cooled for stable operations such as protection of the Peltier element 122 and the like.

A variety of ways may be used for the said cooling, and particularly, an inside surface of the Peltier element 122 may be so configured as to thermally contact the water stored in the water tank 111 of the mist processing unit 110.

This means that the sterilizing water stored in the water tank 111 of the mist processing unit 110 may be used to cool the Peltier element 122.

The thermal contact by the inside surface of the Peltier element 122 with the water stored in the water tank 111 of the mist processing unit 110 may be realized through a first heat dissipation unit 124 of first material that directly contacts the water stored in the water tank 111, and a second heat dissipation unit 123 of second material interposed between the inside surface of the Peltier element 122 and the first heat dissipation unit 124.

In other words, the inside surface of the Peltier element 122 and the water stored in the water tank 111 may be mutually and thermally contacted by at least three (3) mutually different elements.

The first heat dissipation unit 124 may be installed through a hole formed on the water tank 111, where one surface is made to contact the sterilizing water inside the water tank 111 and simultaneously the other surface is made to contact the second heat dissipation unit 123, and a part where the first heat dissipation unit 124 is mounted must be well sealed with an O-ring lest there be any leakage.

The first heat dissipation unit 124 may be disposed at one lateral outside (water tank side) of the second heat dissipation unit 123 to thereby maximize the heat dissipation effect by being contacted with the sterilizing water inside the water tank 111.

The shape of the first heat dissipation unit 124 may not be particularly limited and may be variably formed, and the first heat dissipation unit 124 may take a round shape, a polygonal shape or a star shape. When the first heat dissipation unit 124 is formed therein with a plurality of protruders, the heat dissipation effect can be further enhanced.

Furthermore, a surface where the first heat dissipation unit 124 contacts the sterilizing water of the water tank 111 may have a high possibility of being contaminated due to several ions and the like. Therefore, the first material may be formed with a material that can be less contaminated due to sterilizing water as compared against the second material.

As a detailed example, the first material of the first heat dissipation unit 124 may be so formed as to include SUS material, and the second material of the second heat dissipation unit 123 may be so formed as to include aluminum material. Then, the surface contacted by the sterilizing water of the water tank 111 may be minimized in oxidation.

In order to further improve the heat dissipation function, the second heat dissipation unit 123 may be formed in a water cooling system to accommodate therein a cooling liquid. At this time, the second heat dissipation unit 123 may be formed by including a main body 123-1 accommodating the cooling liquid and a lid 123-2.

Furthermore, the main body 123-1 may be formed therein with several protruding structures to further increase the cooling effect.

The lid 123-2 of the second heat dissipation unit 123 may be so formed with a nut or a bolt as to be fixed to the main body 123-1, and an O-ring may be interposed between the main body 123-1 and the lid 123-2 for tight seal therebetween.

The housing may be disposed with a variety of displaying means for displaying the operations of skin beauty device 100. For example, LED lamps may be disposed for displaying various pieces of information including, but not limited to, power ON/OFF states, operational states of mist processing unit 110. operational states of temperature stimulation processing unit 120, operational states of EP processing unit 130, operational states of light processing unit 140, operational states of vibration processing unit 145, and current cooling/heating set-up states.

The power supply unit 170 may be so configured as to be used for conversion of AC power to DC power, also configured to include a chargeable battery and may be variably configured to include a battery charging circuit if necessary. Various elements including PCBs forming the electric circuits, batteries and the like may be housed at an inside space of the handle part 210.

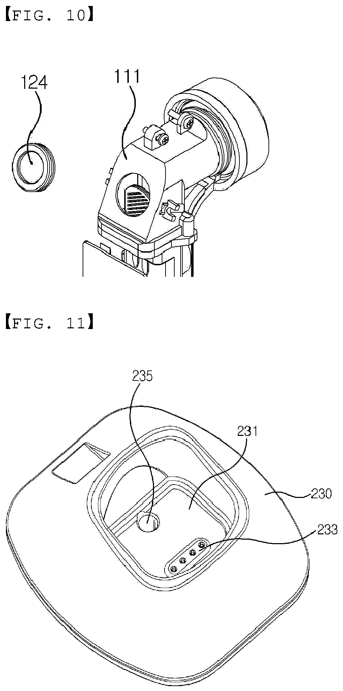

Referring to FIG. 11, the skin beauty device 100 may include a cradle part 230 that plays a role of a rest (holder).

The cradle part 230 may be so formed as to hold a lower end of the handle part 210, and may be so configured as to perform a function of charging a battery. At this time, the handle part 210 and the cradle part 230 may be disposed with a charging terminal 233 so as to be mutually and electrically contacted when the handle part 210 is mounted.

A floor 231 of a rest space of the cradle part 230 may be formed with a drain hole 235 to allow the water flowing down through the handle part 210 to be drained out.

That is, the water may flow down on various elements of handle part 210 including mist function ON/OFF switches 119-2 by being formed in water drops during the course of the mist spraying operations being continuously performed. When the floor of the cradle part (230) is formed with a drain hole 235, the water leaked through the handle part 210 can be flowed to an outside and the risk of being short-circuited by the battery charging terminal 233 can be prevented to thereby enhance the safety.

The controller 150 may perform the role of overall control over the skin beauty device 100 in response to operational commands inputted through the input unit 160.

The methods controlling the skin beauty device 100 by the controller 150 may be variably devised but the present invention is not particularly limited.

For example, the controller 150 may allow the mists to be sprayed by driving the mist processing unit 110 in response to the ON/OFF signals of mist function inputted through the input unit 160, and maintain the skin contact member 121 at an appropriate temperature by driving the temperature stimulation processing unit 120 in response to heating/cooling stimulation set-up signals.

Furthermore, the electric stimulation can be generated through the EP electrodes by driving the EP processing unit 130 in response to the ON/OFF signals of EP function. The controller 150 may control in such a manner that an appropriate light be irradiated through the transparent window 141 by driving the light processing unit 140 in response to the ON/OFF signals of light stimulation function, and that the vibration stimulation be generated by driving the vibration processing unit 145 in response to the ON/OFF signals of the massage vibration function.

Particularly, when a command instructing to implement the skin care method according to the present invention through the input unit 160, the controller 150 may control the skin beauty device 100 in such a manner that a series of skin care functions be continuously implemented.

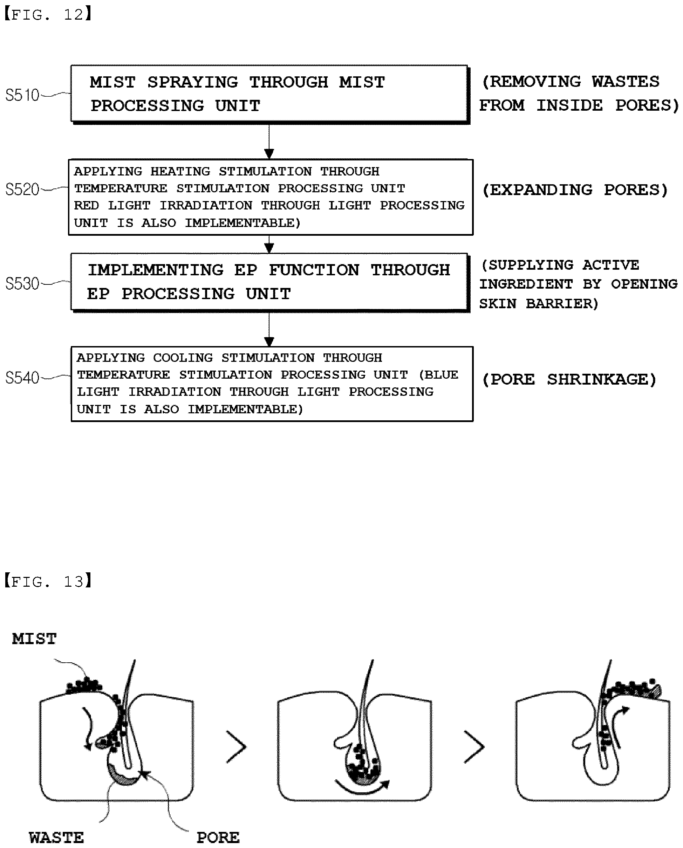

FIG. 12 illustrates a skin care method according to an exemplary embodiment of the present invention, where mist spray, thermal stimulation, EP function implementation and cold (cooling) stimulation application processes are sequentially realized.

First, the controller 150 may control in such a manner that the mist processing unit 110 generates mists for a predetermined time and spray the mists (first step, S510).

The mists sprayed from the mist processing unit 110 may perform two major important roles for the user's skin.

First, the mists can change the user's skin of positive (+) potential to that of negative (-) potential.

When the skin surface of a user is a positive potential (i.e.,: +300mV), only approximately less than 0.3% of effective component from outside can penetrate the skin due to skin barrier. However, because the mists generated by electrolysis may have a polarity of negative potential (i.e.,: -250mV), and when the user's skin is changed to a skin of negative potential by spraying the changed mists to the user, the skin barrier can be reduced.

Second, the mists can discharge wastes from inside the pores.

Referring FIG. 13, the mists generated through the mist processing unit 110 under a state of hydrogen among the water molecules being dissolved and maintained as such may have a size (i.e.,: diameter of smaller than 5nm) of nano bubble property capable of penetrating into pores, and may pump out the sebum and various wastes contained inside the pores using gas pressure.

Cosmetics may be supplied to the user's skin after the first step is finished.

For example, the cosmetics may be supplied to skin after the first step is completed, or may be supplied to the skin before EP function is started at second step or third step, or may be supplied to the skin while the EP function is being implemented, such that the cosmetics may be supplied at an appropriate time, if necessary.

When the first step S510 is completed, the temperature stimulation processing unit 120 may control in such a manner that a heating stimulation is applied for a predetermined time (second step, S520).

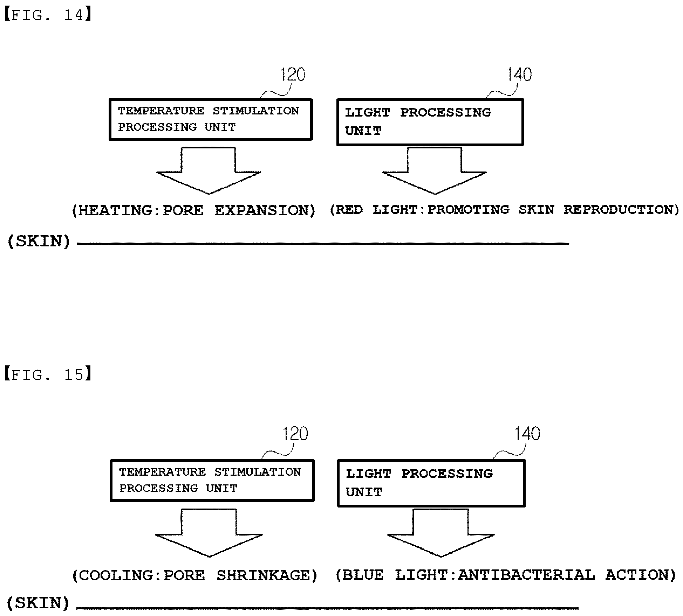

Referring to FIG. 14, when a heating stimulation is applied to the user's skin at second step S520, pores may be expanded and the expanded pores may allow the active ingredient of cosmetics to be well penetrated into the pores.

The second step S520 may be so configured as to allow a light in a particular wavelength band to be applied from at least a part of the heating stimulation-applied period through the light processing unit 140.

Here, the irradiated light may be provided with various purposes including the increase of pore-expanding efficiency or being conducive to skin reproduction, and the wavelength of the light may be adequately determined depending on the purposes thereof.

As a detailed example, the second step S520 serves to expand the pores by supplying an approximate 40 °C of warmth to the user's skin, and simultaneously supplying energy to the user's skin by irradiating a red light to thereby promote the skin reproduction.

When the second step S520 is completed, the EP processing unit 130 may control in such a way that an electric signal is applied to the EP electrodes for a predetermined time (third step, S530).

The EP function implemented by the third step S530 may be to play the role of having the cosmetics to be easily absorbed into the skin by opening the skin barrier.

The third step S530 may be so configured as to apply vibrations to the EP electrodes from at least a part of the electric signal-applied period through the vibration processing unit 145.

As a detailed example, at the third step S530, an electric signal of particular frequency (i.e.,: 10k~40kHz) may be applied to the user's skin to allow the cosmetics to be well absorbed into the skin by opening the skin barrier, and at the same time, vibration stimulation may be applied to the skin to allow the active ingredient of cosmetics to be more efficiently absorbed thereinto.

When the third step S530 is completed, the temperature stimulation processing unit 120 may control in such a manner that a cooling stimulation is applied for a predetermined time (fourth step, S540).

Referring to FIG. 15, the fourth step S540 is a process of shrinking the pores, and may be so configured as to allow a light in a particular wavelength band to be applied from at least a part of cooling stimulation-applied period through the light processing unit 140.

Here, the irradiated light may be provided for various purposes including further increase of pore shrinkage efficiency, and implementation of antibacterial activity, and the light wavelength may be adequately determined depending on the purposes thereof.

As a detailed example, at the fourth step S540, moisturization and maintenance of active ingredients can be promoted by allowing the skin to be quickly cooled up to about 5°C within several seconds to thereby shrink the pores using the cooling stimulation through the temperature stimulation processing unit 120, and antimicrobial treatment may be implemented through blue light irradiation, whereby the skin care processes can be completed.

The time for implementing each step may be variably set up if necessary, may be set up by default, or may be so configured as to allow the user to set up for use.

As one example, the first step S510 may be set up for about one to five minutes, the second step S520 and the third step S530 may be set up for one to four minutes, and the fourth step S540 may be set up for one to two minutes, but the present invention is not limited thereto.

Furthermore, the skin beauty device 100 may vocally guide various pieces of information including, but not limited to, progressive orders of first to fourth step (S510 to S540), user's skin care know-how related to each step, time where each process is being progressed, effects expected therefrom to further increase the conveniences for the users.

The skin care method of skin beauty device according to the present invention may be embodied in a computer program command type implementable through a computer, and may be recorded in the computer-readable media.

The computer-readable media may include all kinds of recording devices in which data readable by a computer system are stored.

Although the present invention has been described and illustrated so far using drawings and explanations based upon exemplary embodiments, it would be apparent to, and readily appreciated by, those of ordinary skill in the art that certain modifications, variations, and alternative constructions would be apparent, while remaining within the spirit and scope of the invention.

### [Description of Reference Numerals]

| | | | |
|---|---|---|---|
| 100: | skin beauty device | 110: | mist processing unit |
| 111: | water tank | 112: | electrode module |
| 113: | ultrasonic vibrator | 117: | mist outlet |
| 119-1: | inlet cap | 120: | temperature stimulation processing unit |
| 121: | skin contact member | 122: | thermoelement (Peltier element) |
| 123: | second heat dissipation unit | 123-1: | main body |
| 123-2: | lid | 124: | first heat dissipation unit |
| 130: | EP processing unit member | 132: | outer electrode member |
| 140: | light processing unit | 141: | transparent window |
| 145: | vibration processing unit | 150: | controller |
| 160: | input unit | 170: | power supply unit |
| 210: | handle part | 220: | head part |
| 221: | first head part | 222: | second head part |
| 230: | cradle part | 233: | battery charging terminal |
| 235: | drain hole | | |

## Claims

1. A skin care method of skin beauty device, the method implemented by the skin care device including a mist processing unit that sprays sterilized water in a mist type by generating the sterilized water through water electrolysis, a temperature stimulation processing unit applying a temperature stimulation, an EP processing unit applying an electric signal to an electroporation electrode, a power supply unit supplying an electric power, an input unit receiving an operation command and a controller performing a control in response to the operation command inputted through the input unit, the method comprising:
controlling in such a manner that the mist processing unit sprays the mist for a predetermined time (first step):
controlling in such a manner that the temperature stimulation processing unit applies heating (thermal) stimulation for a predetermined time (second step);
controlling in such a manner that the EP processing unit applies an electric signal to an electroporation electrode for a predetermined time (third step); and
controlling in such a manner that the temperature stimulation processing unit applies a cooling stimulation for a predetermined time (fourth step).

2. The method of claim 1, wherein the skin beauty device further comprises a light processing unit outputting a light for skin care, and wherein the second step is so configured as to allow the light processing unit to apply a red light in a particular wavelength band from at least a part of the period in which the heating stimulation is applied.

3. The method of claim 1, wherein the skin beauty device further comprises a light processing unit outputting a light for skin care, and wherein the fourth step is so configured as to allow the light processing unit to apply a blue light in a particular wavelength band from at least a part of the period in which the heating stimulation is applied.

4. The method of claim 1, wherein the skin beauty device further comprises a vibration processing unit applying a vibration stimulation to the skin, and wherein the third step is so configured as to allow the vibration processing unit to apply vibrations to the skin from at least a part of the period in which an electric signal is applied to the electroporation electrode.

5. The method of claim 1, wherein the mist processing unit is so configured as to spray the mist having a diameter smaller than 5nm.

6. A computer-readable recording medium recorded with a program for implementing, by a computer, the skin care method of skin beauty device of any one claim in claims 1 to 5.
